# EUROPEAN PATENT APPLICATION

(11) **EP 2 186 537 A1**
(43) Date of publication of application: **19.05.2010**
(21) Application number: 08168679.2
(22) Date of filing: 07.11.2008
(51) Int. Cl.: A61M 15/06

(54) **Inhaler, comprising a hydrogen generator**

(71) Applicant: Inhaleness B.V., 2251 AP Voorschoten (NL)
(72) Inventor: Lugtigheid, Gerardus Wilhelmus, 3206, BW Spijkenisse (NL)
(74) Representative: Lenzing Gerber Stute

(57) **Abstract**

The invention relates to a Soft Mist Inhaler (10) (SMI), comprising:
- a first reservoir (106) for storing a hydrogen containing fluid,
- a second reservoir (107) for storing an activator fluid,
- a gas compartment,
- a fluid connector (110), for connecting the first and the second reservoir with the gas compartment in order to allow the hydrogen containing fluid and the activator fluid to flow to the gas compartment in order to mix and to thereby allow the hydrogen to be released from the hydrogen containing fluid into the gas compartment,
- a pre-catalyst mixing chamber (203),
- a first air inlet, connected to the pre-catalyst mixing chamber in order to allow ambient air to enter the pre-catalyst mixing chamber
- a gas connector (114), for connecting the gas compartment with the pre-catalyst mixing chamber, in order to allow hydrogen to flow from the gas compartment to the pre-catalyst mixing chamber in order to mix with ambient air from the first air inlet,
- a catalyst chamber (212) with a catalyst for converting the fluid contained in the pre-catalyst chamber into a mixture of warm air and water vapor, wherein the catalyst chamber is connected to the pre-catalyst mixing chamber in order to allow the fluid contained in the pre-catalyst mixing chamber to flow past the catalyst,
- a container for storing an active substance, comprising a contact area for exposing the active substance to a flow,
- a guide for guiding the mixture of warm air and water vapor past the contact area of the container, in order for active substance to be taken up by the mixture of warm air and water vapor of a second temperature level and to create an aerosol containing the active substance.
The invention also relates to a system and method for generating hydrogen.

## Description

According to a first aspect, the invention relates to an inhaler, suitable for the administration of active substances to a mammal. According to a second aspect, the invention relates to a system and method for generating hydrogen.

### Technical field

The invention relates in a first aspect to a soft mist inhaler (SMI) for application to the lung. An SMI produces an aerosol containing an active substance for systemic delivery in the lung. The aerosol is released from the SMI and can be inhaled by a user. The aerosol is used as the transport vehicle to carry the active substance into the airways.

### Background of the technology

An SMI typically releases the aerosol from the device at low velocity. This hampers the penetration of the aerosol in the deep lung. In order to improve the operation of an SMI it is important that the aerosol is administered early during the inhalation. By dosing the aerosol at the start of the inhalation, optimum use is made of the kinetic energy of the breathing flow to carry the aerosol into the lung as deep as possible.

The required particle size of an aerosol containing an active substance is determined by its target area in the airways. Systemic actives must be delivered to the alveoli and the particle size must therefore be smaller then in case local actives are delivered at a specific area in the higher airways.

The actual particle size for the aerosol is depended on its temperature. That means that controlling the temperature of the aerosol in the SMI is most important in order to be able to control the particle size of the aerosol that will be released by the SMI.

The active substance to be delivered by means of the SMI is contained in the aerosol. That means that when manipulating the aerosol in order to control the particle size, care should be taken that the temperature doesn't exceed a maximum temperature, depending on the active substance, in order not to risk deteriorating of the active substance.

### Summary of the invention

According to one aspect of the invention there is provided an inhaler, comprising:
- a first reservoir for storing a hydrogen containing fluid,
- a second reservoir for storing an activator fluid,
- a gas compartment,
- a fluid connector, for connecting the first and the second reservoir with the gas compartment in order to allow the hydrogen containing fluid and the activator fluid to flow to the gas compartment in order to mix and to thereby allow the hydrogen to be released from the hydrogen containing fluid into the gas compartment,
- a pre-catalyst mixing chamber,
- a first air inlet, connected to the pre-catalyst mixing chamber in order to allow ambient air to enter the pre-catalyst mixing chamber
- a gas connector, for connecting the gas compartment with the pre-catalyst mixing chamber, in order to allow hydrogen to flow from the gas compartment to the pre-catalyst mixing chamber in order to mix with ambient air from the first air inlet,
- a catalyst chamber with a catalyst for converting the fluid contained in the pre-catalyst chamber into a mixture of warm air and water vapor, wherein the catalyst chamber is connected to the pre-catalyst mixing chamber in order to allow the fluid contained in the pre-catalyst mixing chamber to flow past the catalyst,
- a container for storing an active substance, comprising a contact area for exposing the active substance to a flow,
- a guide for guiding the mixture of warm air and water vapor of a second temperature level past the contact area of the container, in order for active substance to be taken up by the mixture of warm air and water vapor of a second temperature level and to create an aerosol containing the active substance.

According to one preferred embodiment of the invention the inhaler comprises means for controlling the temperature level of the mixture of warm air and water vapor. Controlling the temperature level of the mixture of warm air and water damp is favorable - on the one side - in order to achieve a temperature level which is comfortable to inhale and - on the other side - to control the particle size of the aerosol.

The temperature level of the mixture of warm air and water vapor exiting the catalyst chamber can be easily controlled when the means for controlling the temperature level of the mixture of warm air and water vapor comprises:
- a post-catalyst mixing chamber for mixing the warm air and water vapor of a first temperature level formed in the catalyst chamber with ambient air, in order to form a mixture of warm air and water vapor of a second temperature level, wherein the post-catalyst mixing chamber is provided with a second air inlet to allow air to enter the post-catalyst mixing chamber.

According to another preferred embodiment of the invention the inhaler comprises:
- a first element with the first reservoir for storing a hydrogen containing fluid, the second reservoir for storing an activator fluid, the gas compartment, and the fluid connector, for connecting the first and the second reservoir with the gas compartment,
- a second element adapted to be connected to the first element, with the pre-catalyst mixing chamber, the gas connector, for connecting the gas compartment with the pre-catalyst mixing chamber, the catalyst chamber with a catalyst for converting the fluid contained in the pre-catalyst chamber into a mixture of warm air and water vapor of a first temperature level, and the post-catalyst mixing chamber for mixing the warm air and water vapor of a first temperature level
formed in the catalyst chamber with ambient air, in order to form a mixture of warm air and water vapor of a second temperature level,
- a third element, adapted to be connected to the second element, with the container for storing an active substance, comprising a contact area for exposing the active substance to a flow, the guide connected with the post-catalyst mixing chamber for guiding the mixture of warm air and water vapor of a second temperature level from the post-catalyst mixing chamber along the contact area of the container.

The advantage of this embodiment is that the first, second and third element can be made interchangeable. That means that in case one of the elements has reached the end of its lifetime, this element can be replaced, while the other elements can continue to be used.

The first and the third elements are likely to have a shorter lifetime then the second element. The fact that the elements can be replaced means that the operational costs of the inhaler according to the invention can be reduced.

According to a preferred embodiment of the invention, at least one of the first and second reservoirs for storing a hydrogen containing fluid or for storing an activator fluid are formed as a bag from an elastic material, wherein the interior of the bag is suitable for exerting a pressure on the fluid contained in the reservoir.

The advantage of this embodiment is that a simple and reliable way of disposing the fluids from their respective containers is ensured.

According to a preferred embodiment of the invention, the bag from an elastic material is adapted to put a pressure on the fluid contained in the reservoir of 0.2 - 0.5 Bar.

According to a preferred embodiment of the invention, both the first and second reservoirs are formed as a bag from an elastic material, suitable for exerting a pressure on the fluid contained in the reservoir, wherein the assembly of both reservoirs is contained in a further bag from an elastic material in order to provide an equal pressure on the exterior of both reservoirs.

According to a preferred embodiment of the invention, the fluid connector, for connecting the first and the second reservoir with the gas compartment is provided with a flow regulator in order to control the amount of hydrogen containing fluid and activator fluid entering the gas compartment.

According to a preferred embodiment of the invention, the flow regulator is exposed to both ambient pressure and the pressure inside the gas compartment, wherein the opening and closing of the flow regulator is controlled by the difference in pressure between the ambient and the pressure inside the gas compartment.

The flow regulator will be used to produce the necessary amount of hydrogen in the gas compartment. As soon as the pressure in the gas compartment exerts a maximum threshold, the pressure difference between the pressure of the hydrogen inside the gas compartment and the ambient air pressure will move the flow regulator towards its closed position.

According to a preferred embodiment of the invention the first and second reservoir are positioned inside the gas compartment, wherein the gas compartment is provided with an outlet opening, and wherein the outlet opening on one end is connected to the gas connector for connecting the gas compartment with the pre-catalyst mixing chamber, and wherein the outlet opening on the other end is connected to a gas channel in the form of a perforated tube, wherein the gas channel supports the first and second reservoirs and creates a channel between the two, wherein the perforations allow the gas in the gas compartment to enter the interior of the gas channel, in order to flow towards the outlet opening.

The presence of the gas channel will enable the gas to find its way towards the outlet opening and will avoid this outlet opening to be obstructed by any element present in the gas compartment.

According to a preferred embodiment of the invention, the gas connector for connecting the gas compartment with the pre-catalyst mixing chamber is provided with a moveable stopper for controlling the amount of gas entering the pre-catalyst mixing chamber, wherein the moveable stopper is biased towards a closed position by the action of a spring and wherein the moveable stopper can be displaced towards it's open position by means of an under pressure, created at the downstream end of the moveable stopper.

The presence of this moveable stopper allows the inhaler according to the invention to be breath-actuated. The position of the moveable stopper can be influenced by exerting a suction pressure on the mouthpiece of the inhaler.

According to a preferred embodiment of the invention, the container for storing an active substance comprises an absorbent element for absorbing the active substance and a spring in order to exert a pressure on the absorbent element in order to forward the active substance towards the surface of the absorbent element.

This provides a redundant and simple solution for ensuring that the active substance can be taken up by the flow of air and water vapor flowing along the exterior of the absorbent element.

### Brief description of the drawings

Reference will now be made, by way of example, to the accompanying drawings, in which:
Figure 1 is a perspective view of an embodiment of the inhaler of the invention,
Figure 2 shows the cartridge, the diffuser and the capsule of the inhaler according to fig. 1,
Figure 3 is a cross sectional view of the inhaler according to fig. 1,
Figure 4 is a first cross sectional view of the cartridge of the inhaler according to fig. 1,
Figure 5 is a second cross sectional view of the cartridge of the inhaler according to fig. 1,
Figure 6 shows the diffuser according to figure 3 in a perspective view, wherein the outer shell of the diffuser is shown as a transparent layer in order to show piston inside the diffuser, in a first or non-breath activated position,
Figure 7 shows the diffuser according to figure 3 in a perspective view, wherein the outer shell of the diffuser is shown as a transparent layer in order to show piston inside the diffuser, in a second or breath activated position,
Figure 8 shows part of the outer shell of the diffuser according to fig. 3, wherein the air inlet of the pre-catalyst chamber on the inside is closed off by means of the piston,
Figure 9 shows part of the outer shell of the diffuser according to fig. 3, wherein the air inlet of the pre-catalyst chamber on the inside is open,
Figure 10 shows in perspective view the catalyst of the diffuser according to fig. 3,
Figure 11 shows in an exploded view the components of the capsule of the inhaler according to fig.1,
Figure 12 is a cross sectional view of the capsule according to figure 11, with a sleeve in order to allow the capsule to be stored,
Figure 13 shows the capsule according to figure 12 without the sleeve, in order to allow the capsule to be used, and
Figure 14 shows the capsule according to figure 12 and 13 after its use.

### Detailed description of the invention

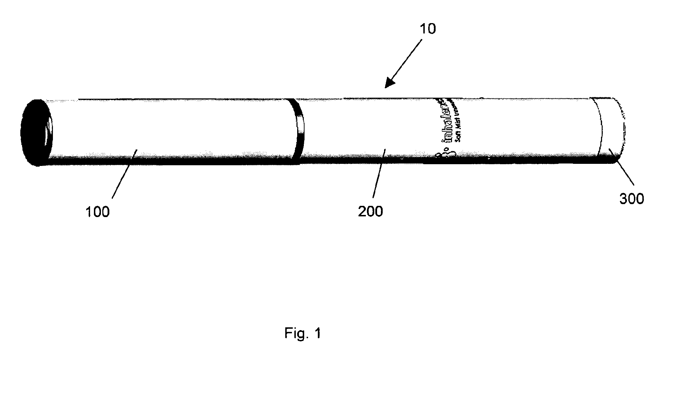

Fig. 1 is a perspective view of an embodiment of the inhaler 10 of the invention. Fig.1 shows that the inhaler 10 has the form of an elongated cylindrical tube. The inhaler 10 is adapted to create an aerosol inside the apparatus and to release the aerosol via an outlet opening. The inhaler 10 according to fig. 1 is breath-actuated. That means that the process of creating and releasing the aerosol is initiated by a breathing action of a user.

The outside dimensions of the inhaler 10 are similar to the outside dimensions of a cigarette. The fact that dimensions of the inhaler 10 are similar to those of a cigarette means that a user can put the inhaler 10 in his mouth. The inhaler 10 can be used without the need of an additional mouthpiece.

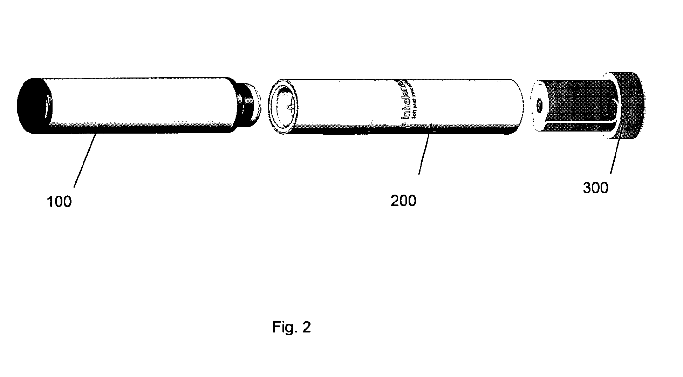

Fig. 2 shows the different elements of the inhaler 10 of figure 1. The inhaler 10 comprises three elements.
At the left hand side a cartridge 100 is shown. The cartridge 100 serves to produce a quantity of hydrogen in its gaseous form. To this end the cartridge 100 contains a hydrogen-containing fluid and an activator. Both fluids can be mixed inside the cartridge 100. The mixing of the fluids will produce the hydrogen in its gaseous form.

The second element of the inhaler 10 according to fig. 1 is a diffuser 200. The diffuser 200 is tightly connected to one end of the cartridge 100. The hydrogen produced inside the cartridge 100 is transferred to the diffuser 200 in order to create warm air and water vapor inside the diffuser 200. The diffuser 200 is provided with a catalyst. The diffuser 200 is adapted to allow the hydrogen to be mixed with ambient air upstream of the catalyst. This mixture is, by means of the catalyst, converted into warm air and water vapor. This warm air and water vapor form an aerosol of water droplets.

The heat produced during this conversion is contained in the air and water vapor. That means that the air and water vapor are relatively warm. Downstream of the catalyst the diffuser 200 is provided with a further air inlet in order to allow the air and water downstream of the catalyst to mix with ambient air. By means of this mixing step the temperature of the air and water vapor can be controlled and decreased in order to make them suitable for inhalation and deposition on the target area in the airways or the deep lung.

The third element of the inhaler is a capsule 300. The capsule is positioned downstream from the catalyst of the diffuser 200. The capsule 300 contains an active substance. The active substance is stored in a container with a contact area. This contact area is adapted to allow direct contact between the active substance contained in the capsule and the warm air and water vapor. This direct contact allows the warm air and water vapor to take up active substance in order to create an aerosol containing the active substance.

The mixture of the aerosol and active substance is released from the capsule via an outlet and can be inhaled by a user.

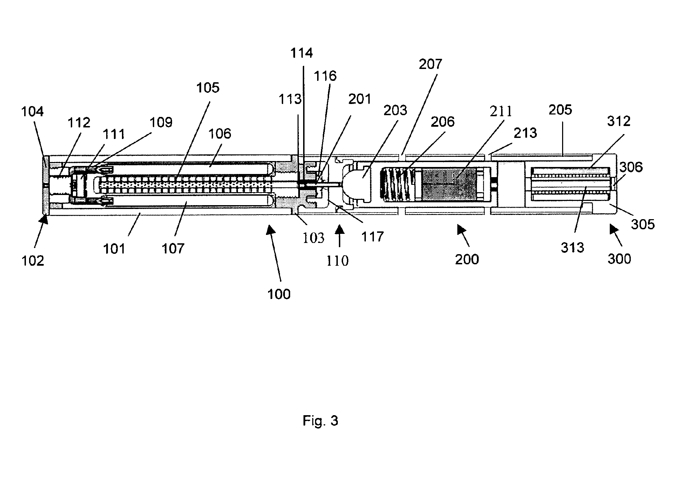

Fig. 3 is a cross sectional view of the inhaler according to fig. 1. Fig. 3 shows that the cartridge 100 is provided with a fluid tight outer shell 101. The free end 102 of the cartridge 100 is sealed off with a cap 104. In the wall of the cap 104 an aperture 108 is present. The other end 103 of the cartridge 100 is provided with a connector 110 for connecting the cartridge to the diffuser 200.

The core of the cartridge 100 is formed by a gas channel 105. This gas channel 105 has the form of a perforated tube. The space between the inside of the outer shell 101 and the outside of the gas channel 105 is filled by means of a first 106 and a second 107 reservoir, which have the form of flexible bags. These bags may be oversized in order to control the pressure on the weld seams of the bags.

The first reservoir 106 is adapted to store a hydrogen containing fluid. This fluid contains, for example, an aqueous metal hydrate, such as a borohydrate salt. The use of such an aqueous metal hydrate provides a relatively cheap and safe means for storing a quantity of hydrogen inside the inhaler 10. Since the hydrogen is stored in a fluid, solid state, there is no need for a pressure vessel inside the inhaler 10 in order to store the hydrogen.

The first reservoir 106 is preferably a flexible bag made of plastics, elastomers or other materials that are generally deformable and capable of containing fluid solutions.

In a first embodiment the fuel is contained inside the reservoir 106 under pressure. That means that the wall of the reservoir 106 exerts a pressure on the fluid inside the bag in order to facilitate the dispensing of the fuel from the outlet opening of the first reservoir.

Alternatively, the reservoir 106 operates in cooperation with a mechanical element, like a spring, in order to exert a pressure on the outside of the wall of the reservoir and in order to facilitate the release of fuel from the reservoir 106 by this mechanical action.

The second reservoir 107 is adapted to store an activator. In the context of this invention, the word 'activator is used' to indicate a fluid which is capable of mixing with a hydrogen containing fluid, such as an aqueous metal hydrate, in order to initiate the release of the hydrogen from the mixture. The activator to be used with borohydride may contain acetic acid.

Similar to the first reservoir 106, the second reservoir 107 may contain the fluid under pressure. This facilitates the driving out of the activator from an outlet of the second reservoir 107. Alternatively the reservoir 107 is operated in cooperation with a mechanical element, like a spring, in order to release fuel from the reservoir 107.

According to a preferred embodiment, an elastic cover (not shown) is provided in order to contain both the reservoir 106 and 107. The presence of the additional bag provides an equal tension on the exterior of both reservoirs 106 and 107. That means that an equal pressure is maintained inside both reservoirs 106 and 107. Due to the symmetric form of the outlets of both reservoirs 106 and 107, an equal mixing flow from both reservoirs 106 and 107 to the mixing chamber is provided.

The first 106 and second 107 reservoirs have an outlet for releasing the contained fluid. These outlets are both connected to a mixing valve 109. The mixing valve is used to allow the fuel and activator to be mixed.

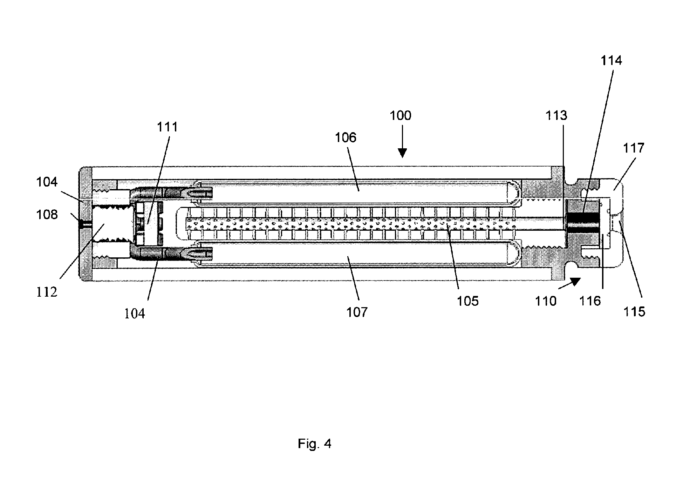

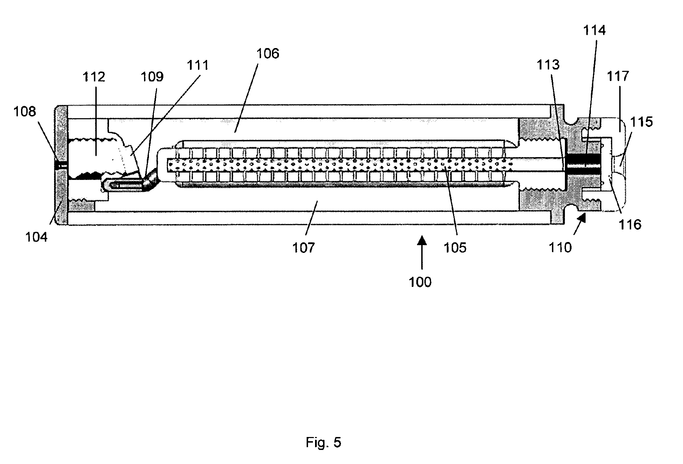

The mixing valve 109 is provided with a flow regulator 111. The flow regulator is connected to a bellow 112. The functioning of the mixing valve 111 is explained with reference to figs. 4 and 5.

Figure 4 shows the first cross sectional view of the cartridge 100 of the inhaler 10 according to fig. 3. Fig. 4 shows a top view of the mixing valve 109.
Figure 5 is a second cross sectional view of the cartridge 100 of the inhaler 10 according to fig. 1. Fig. 5 shows the mixing valve 109 in side view.

The flow of fluid from reservoir 106 and activator from reservoir 107 is controlled by means of the flow regulator 111. The flow regulator 111 can move from a first or open position, as shown in figs. 4 and 5, towards the cap 104 in order to reach a second or closed position (not shown). The position of the flow regulator 111 depends on the pressure exerted on both sides of the flow regulator 111. The flow regulator 111 is connected to the cap 104 by means of a bellow 112. The inside of the bellow 112 is connected to the inside wall of the cap, around the aperture 108. The elastic energy stored in the bellow 112 pushes the flow regulator 111 towards its open position. To ensure the working of the flow regulator 111 the surface of the bellow 112 is directly connected to the inside of the flow regulator 111. This connection will allow the pressure difference between the ambient and the inside of the cartridge 100 to act over the entire contact surface area of the flow regulator 111.

On its opposite side the flow regulator is exposed to the pressure inside the cartridge 100. As soon as the pressure inside the cartridge 100 is high enough to exceed the pressure exerted by the bellow 112, the flow regulator will move towards its closed position.

The hinge of 111 has the function of a spring of a pressure reducer. The flexibility of the hinge can be used to tune the opening and closing of the flow regulator 11. The tighter the hinge will be the more pressure difference will be needed to move the flow regulator 111 between its open a closed positions.

With the flow regulator 111 in its open position the fuel and the activator will be pushed out of the reservoirs 106, 107. The fluids will mix and the mixing will allow the hydrogen in the fuel instantaneous to be freed. The hydrogen gas entering the cartridge will increase the pressure inside the cartridge 100. This will increase the pressure on the flow regulator 111, which as a result will be pushed towards its closed position and the flow of fluid and activator will be stopped. In this way automatically a certain level of hydrogen pressure is obtained, without exceeding a maximum pressure inside the cartridge 100.

The hydrogen created inside the cartridge 100 will enter the gas channel 105 and will flow towards the outlet 115 in the connector 110 of the cartridge 100. At the end of the gas channel 105 a membrane 113 is used to close off the tube of the gas channel 105. The hydrogen gas is able to flow through the membrane 113. Any liquid is not able to pass through the membrane 113. That means that the membrane 113 is able to avoid liquid from leaving the cartridge 100 and flowing towards the diffuser 200.

Downstream of the membrane 113 the gas is guided through a channel 114 in the connector 110 towards the outlet opening 115. In between the channel 114 and the outlet opening a septum 116 is positioned. This septum 116 is used, in combination with a needle attached to the diffuser, to create a gas tight connection between the cartridge 100 and the diffuser 200. The outlet opening 115 is formed in the cap 117. The septum 116 is fixed at its position by means of the cap 117.

The hydrogen produced inside the cartridge 100 will be kept under pressure inside the cartridge 100 until the gas is able to enter the diffuser 200, by opening an inlet opening of the diffuser 200. That means that the cartridge 100 forms a gas accumulator.

The cartridge 100 is essentially a disposable. As soon as the cartridge 100 is no longer able to produce hydrogen, the cartridge 100 needs to be replaced.
A window can be placed in the shell 101 in order to allow visual monitoring of the amount of fuel and / or activator in the cartridge 100.

Returning to fig. 3 the cartridge 100 is connected to the diffuser 200 by means of the connector 110 of the cartridge 100. The connector 110 of the cartridge 100 cooperates with an annular, protruding ridge 210 of the diffuser 200. The ridge 210 is adapted to be snapped onto the connector 110 by means of a snap-lock connection.

The outside of the diffuser 200 is formed by a gas-tight shell 205. The end of the diffuser adapted to be snapped on the cartridge 100 is provided with a needle 201. The needle 201 is positioned on the central axis of the diffuser 200 and is adapted to enter the outlet opening 115 of the cartridge 100.

When positioning the diffuser 200 on the cartridge 100 the needle 201 will pierce through the septum 116. The septum 116 and the needle 201 guarantee a gas-tight connection between the two elements 100, 200.

The diffuser 200 has a pre-catalyst mixing chamber 202 in order to receive any hydrogen gas flowing out of the cartridge 100 in the diffuser 200. The connection between needle 201, guiding the gas, and the inlet of the pre-catalyst 202 mixing chamber is closed off by means of a piston 203 with a flexible tip.

The piston 203 is positioned inside the diffuser 200 in order to be able to move with respect to the shell 210 and all other components of the diffuser 200. The piston 203 is held in a first or closed position by means of a spring 206. The spring pushed the piston 203 against the end of the needle 201 in order to avoid hydrogen entering the diffuser 200.

The piston can move from its first or closed position towards a second or open position. In this open position the inlet of the pre-catalyst 202 mixing chamber is no longer closed and hydrogen produced in the cartridge 100 will enter the diffuser 200.

The movement of the piston 203 from its closed towards its open position is controlled by the suction force exerted by a user on the inhaler 10. The suction force of the user will generate an under pressure by means whereof the piston 203 is moved. That means that the inlet of hydrogen into the diffuser is breath controlled.

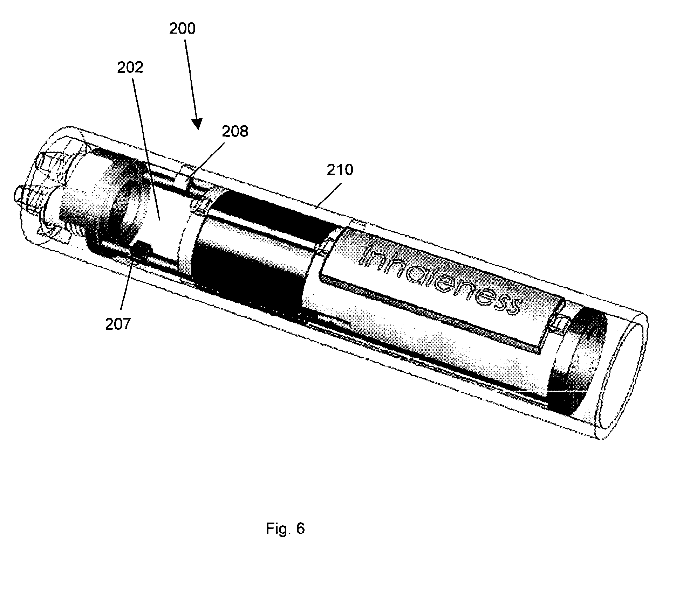

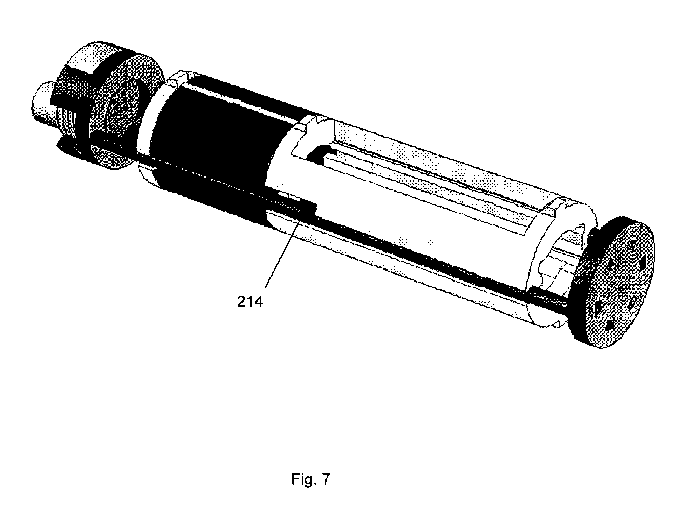

The piston 203 has multiple functions in the diffuser 200. The position of the piston 203 is also used in order to enable air to be mixed with the hydrogen received in the pre-catalyst mixing chamber 202. The functioning of this mixing step is shown in fig. 6 and 7.

Figure 6 shows the diffuser 200 according to figure 3 in a perspective view, wherein the outer shell 210 of the diffuser 200 is shown as a transparent layer in order to show the inside the diffuser 200.

Fig. 6 shows that the pre-catalyst mixing chamber 202 is provided with air inlets 207. These air inlets 207 are on the interior of the shell 210 closed off by elements 208, which are part of the piston 203. As soon as the piston is displaced from its closed position towards its open position, the air inlets 207 are opened and air can enter the pre-catalyst mixing chamber 202. This is shown in fig. 7.

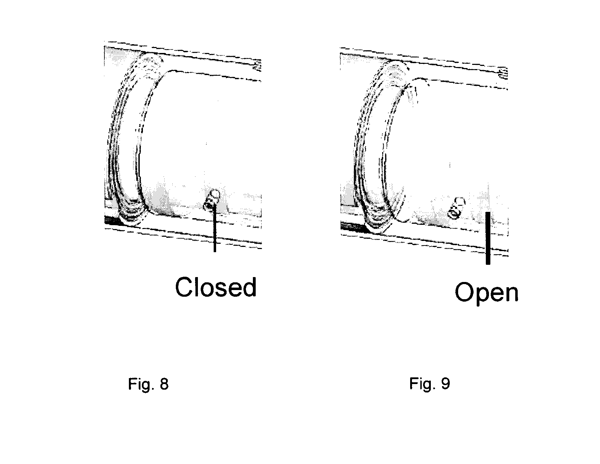

Figure 8 shows part of the outer shell of the diffuser according to fig. 3, wherein the air inlet of the pre-catalyst mixing chamber 202 on the inside is closed off by means of the piston 203. Figure 9 shows part of the outer shell of the diffuser according to fig. 3, wherein the air inlet of the pre-catalyst mixing chamber 202 on the inside is open.

The movement of the piston 203 and the underpressure inside the pre-catalyst 202 mixing chamber are both breath-actuated. That means that the mixing of the hydrogen and the air in the pre-catalyst mixing chamber 202 are automatic.

Returning to fig. 3, the diffuser 200 is further provided with a catalyst, or catalytic converter 211. This catalyst 211 is adapted to convert the mixture of air and hydrogen produced in the pre-catalyst mixing chamber 202 into air, water vapor and heat. The air and the water vapor are received in a post-catalyst mixing chamber, schematically indicated with reference number 212.

The heat produced during the conversion of hydrogen and air into warm air and water vapor is contained in the air and the water vapor. In order to be able to inhale the warm air and the water vapor, the temperature of the warm air and water vapor should be controlled and decreased. To this effect, the post-catalyst mixing chamber 212 is, similar to the pre-catalyst mixing chamber 202, provided with air inlets 213.

Similar to the air inlets 207, the air inlets 213 of the post-catalyst mixing chamber 212 are closed off, at the inside of the shell 210, by the piston 203. The piston 203 is provided with elements 214 in order to close of the air inlets 213 in the first or closed position of the piston 203. As soon as the piston 203 moves towards its open position, the air inlets 213 are opened and air can enter the post-catalyst chamber 212.

The mixing of ambient air with the air and water vapor in the post-catalyst chamber 212 is very important to control the aerosol. The actual particle size of the aerosol is dependent on the temperature of the creation of the aerosol. That means that controlling the temperature of the aerosol in the post-catalyst chamber 212 is most important to control the particle size of the aerosol that will be released by the inhaler 10.

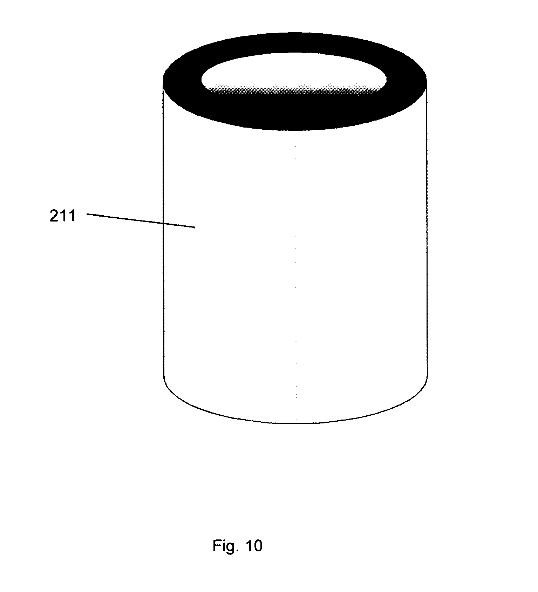

Figure 10 shows in perspective view the catalyst 211 of the diffuser 200 according to fig. 3. Fig. 10 shows that the exterior of the catalyst 211 has the form of an ellipse. This is advantageous in order to use the largest possible volume for the catalyst 211, while at the same time room is freed for the piston 203 on opposite ends of the catalyst 211.

The catalyst 211 has an outside shield, filled with catalyst material in the form of small spheres (size 1 mm). This form is used for the catalyst material in order to create a maximum contact service between the catalyst material and the hydrogen air mixture that flows past the catalyst material. The outside shell of the catalytic converter 211 is on opposite sides closed off with an iron mesh wire, in order to keep the spheres in place, without blocking the airflow through the catalyst converter.

The spheres of catalytic material contain Pt, with a Pt percentage of around 5% in weight, and Pd, with a Pd percentage of around 5% in weight.

According to a preferred embodiment, the upstream end of the catalyst converter comprises substantially Pt containing spheres. This will effect in having an upstream area of the catalyst converter which can be used as starter in order to quickly raise the temperature of the catalytic converter and the gas mixture that flows through the catalytic converter. The downstream part of the catalytic may contain substantially Pd containing spheres.

The third element of the inhaler 10 according to the invention is the capsule 300. Returning to fig. 3 it is shown that the capsule is positioned in between the post-catalyst mixing chamber 212 and an outlet opening 306.

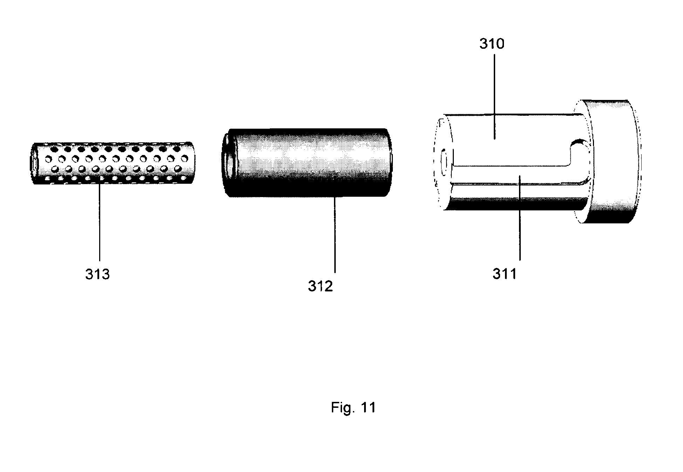

Figure 11 shows in an exploded view the several components of the capsule 300. On the outside the capsule is provided with a shell 310. The shell is at its outside surface provided with a groove 311 in order to assemble the capsule 300 with the diffuser 200.

According to the embodiment of fig. 11 the capsule is mounted in the diffuser 200 by a combined movement of forwarding the capsule in the end part of the diffuser 200 and by rotating the capsule 300 around its axis in order to fix the capsule 300 in the diffuser 200.

In the capsule 300 a container 312 is present in the form of a bag. The bag 312 is capable of storing an active substance. The bag is positioned around the exterior of an elongated spring 313. According to fig. 11 the spring has the form of a regular spring.

The inside of the spring 313 forms a guide for guiding the aerosol from the post-catalyst mixing chamber 212 towards an outlet opening 306 at the end face of the capsule 300. Shell 312 of the capsule can be provided with a window in order to allow for visual inspection of the interior of the capsule 300.
The spring 313 exerts a mechanical force on the inside of the container 312. The exposed contact area of the container 312, that means the contact area that comes into contact with the spring 313, has the form of a membrane. The force exerted by the spring 313 guarantees that the membrane is saturated with active substance.

In use the aerosol will pass along this saturated contact area. In direct contact with the membrane, the relatively warm aerosol will be able to take up active substance. The aerosol leaving the capsule 300 will therefore contain active substance.

The aerosol is passed through the capsule 300 towards an outlet opening 306 in order to be released there from and to be inhaled by a user.

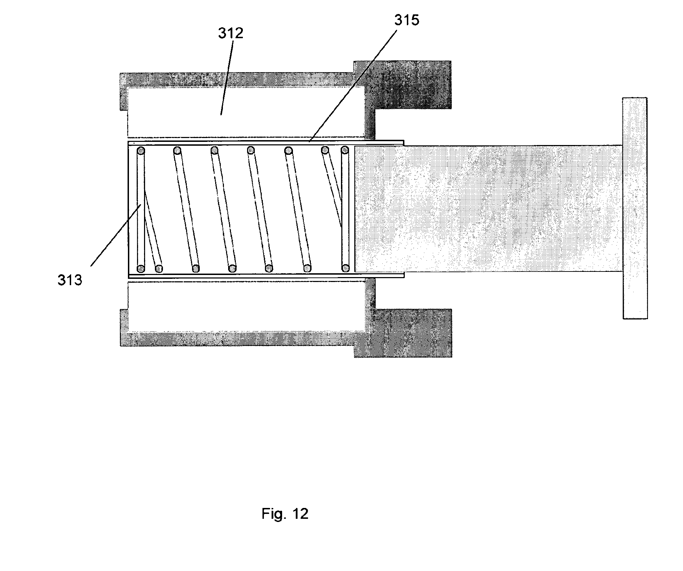

Figure 12 is a cross sectional view of the capsule 300 according to figure 11, with a sleeve 315 in order to allow the capsule 300 to be stored. The sleeve will prevent the spring to exert a force on the container 312. This means that the active substance will not be urged towards the contact area on the inside of the capsule 300.

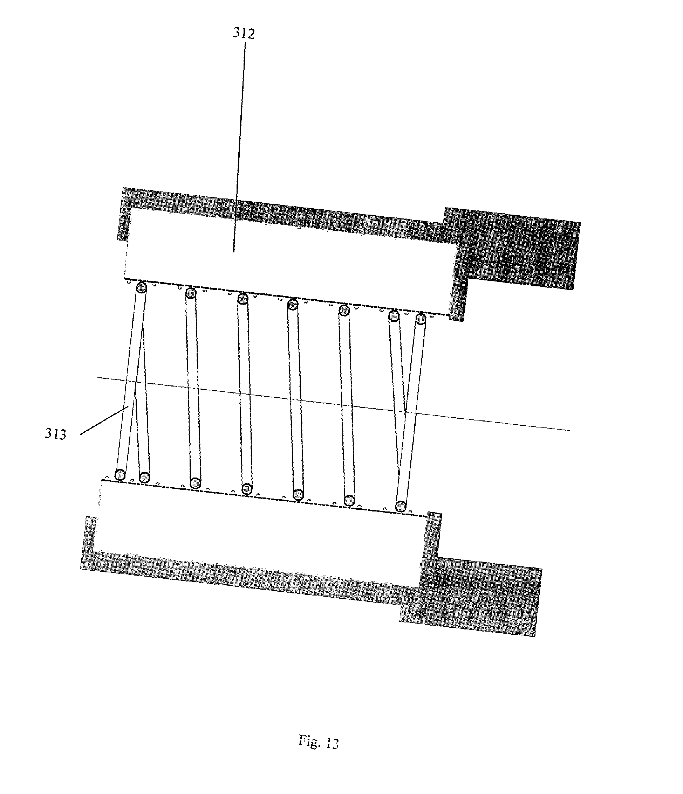

Figure 13 shows the capsule 300 according to figure 12 without the sleeve. This means that the spring 313 will exert a force on the inside of the container and will help the active substance to saturate the interface container-airflow.

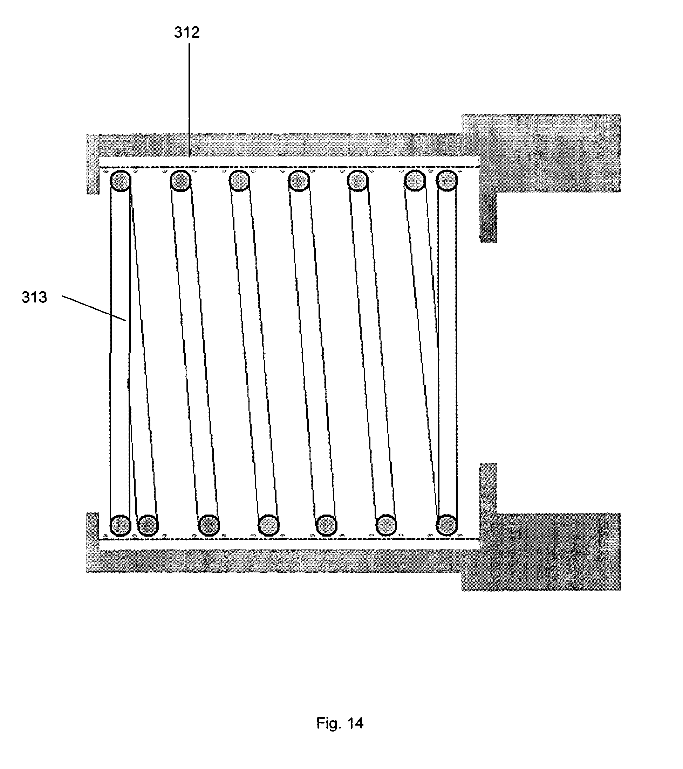

Figure 14 shows the capsule according to figure 12 and 13 after its use. The capsule 300 is a disposable. As soon as the capsule 300 is no longer able to deliver active substance to the aerosol, capsule needs to be replaced.

In the present text reference is made to the production of hydrogen by mixing a first hydrogen containing fluid, with a second fluid or activator for releasing the hydrogen. As described with reference to fig.3 the first reservoir 106 is adapted to store such a hydrogen containing fluid. This fluid contains, for example, an aqueous metal hydride, such as a borohydride salt. The second reservoir 107 is adapted to store the activator.

Below several examples will be described of the method for storing, in a first step, hydrogen in a fluid, such as an aqueous metal hydride, and for releasing the hydrogen, in a second step, by mixing the hydrogen containing fluid with an activator. It should be understood that the method described in the present text can be used to release hydrogen in an inhaler. However the method can be equally be used for other processes for which the storing and releasing of hydrogen is needed.

Table 1 shows a series of hydrogen containing substances which could be used to produce a hydrogen fluid for the method according to the invention. In Table 1 the name of the substances is provided with the molecular weight (MW), the H₂O weight ratio and the H₂ weight percentage of substance including water.

It is the objective to provide an affordable, low pressure, stand alone source of hydrogen, using one of the substances according to Table 1, wherein the resulting fluid has a long shelf life and wherein the fluid provides a high reactivity in the presence of an activator.

A further objective is to provide a system and method which provides a controlled mechanism for releasing hydrogen.

According to a first example the hydrogen containing fluid comprises (in wt.-%): 30% NaBH₄, 1 - 2 % NaOH, and water. The NaBH₄ is mixed with water in order to obtain a liquid. It is necessary to add NaOH in order to obtain a stable mixture and in order to avoid that the NaBH₄ reacts with the water before the releasing of the hydrogen is desired.

According to the literature, in order to obtain a stable mixture of NaBH₄ and water, the amount of NaOH needed in a mixture containing 5 - 50 % NaBH₄ is relatively high, in the order of 5 - 40 %.

According to a second example the hydrogen containing fluid comprises: 60% NaBH₄, 35 - 40% mineral oil and 0 - 5% surfactant. The surfactant is added in order to obtain a stable mixture and in order to avoid that the NaBH4 separates from the mineral oil.

The applicant has observed that a 30% aqueous sodium borohydride solutions, comprising 0.5 - 5 % NaOH, preferably 1 - 2% NaOH produces no measurable quantities of hydrogen. It appears that this solution does have a relatively long shelf life, without the risk of loosing important amounts of hydrogen.

In case 20 - 50 % aqueous sodium borohydride solutions are used, similarly those solutions could comprise 0.5 - 5 % NaOH, preferably 1 - 2% NaOH without producing measurable quantities of hydrogen.

One mechanism that allows for the relatively low amounts of NaOH is the stabilizing effect of NaOH. This stabilizing effect is self-strengthening by the decomposition reaction of borohydride:

NaBH₄ + 2 H₂O → 4 H₂ + NaBO₂

The consumption of water and the formation of sodium metaborate cause the pH of the solution to increase. As a result the system will automatically be stabilized and will less likely decompose.

The stabilized borohydride solution described above may lose less than 25 % of its activity in a period of 12 months.

It is noted that borohydride solutions prepared with distilled water show much more reactivity than borohydride solutions prepared with tap water. This is probably due to the stabilizing effect of certain minerals present in tap water e.g. calcium and magnesium.

The activator that is used in combination with a hydrogen containing fluid according to the invention may contain acetic acid. The acetic acid will neutralize the alkali in the hydrogen containing fluid and allows for the NaBH4 to react with the water to thereby release the hydrogen. Since during the release of hydrogen new alkali will be formed the amount of available acetic acid should be enough to destabilize the hydrogen containing fluid and to continuously neutralize newly formed alkali.

The applicant has also observed that the addition of a mineral acid reduces the pH and accelerates the hydrogen generation. At a pH of 7, it takes approximately 10 seconds to complete 90% of the hydrogen generation reaction. By lowering the pH by 1 unit, the reaction accelerates by a factor of 10, so at a pH of 6, the hydrogen generation reaction is 90% complete in 1 second.
For a system that must be actuated and controlled within 1 second, the preferred pH range therefore is 5 - 6.

All substances mentioned in Table 1, except for Al(BH₄)₃, are solids. In order to easily meter and dispense those substances, the substances should be mixed with a liquid, such as water to produce a stable mixture. Alternatively, the substance could be dispersed in a mineral oil.

In the specific case of using the method and the system according to the invention for inhalation techniques it should be noted that inhalation of acidic aerosols with a pH value of 2 does not harm the lungs (Hickey (ed.), Inhalation Aerosols, p. 133) and for that reason an acid solution with a pH value of approximately 2 has been selected. Acetic acid appears to be very suitable in that respect and has proven to accelerate the hydrogen generation from a borohydride solution. The resulting liquid is slightly acidic.

For inhalation purposes, the borohydride solution, preferably comprises 30 - 40% NaBH4, 1 - 5% NaOH and 69 - 55% water. The acid accelerator solution comprises 40 - 65% acetic acid and 60 - 35% water.
The energy content of a 30% borohydride solution per liter is in excess of 4 MJ. The solutions are preferably stored in similar storage vessels, which are slightly pressurized in order to drive the liquids from the vessel. Mixing is preferably performed in a very small reaction chamber, more preferably in a dual mixing valve that meters and mixes equal volumes of borohydride solution and acid accelerator solution. The reaction forms hydrogen gas instantly.

NaBH₄ + 2 H₂O + CH₃COOH → 4 H₂ + HBO₂ + CH₃COONa

The mixed liquids are preferably retained in a closed container, such that the hydrogen generated builds up a certain pressure. When the hydrogen pressure reaches a certain maximum value, the dual mixing valve will be closed by a lever that is operated by the pressure difference between the container and ambient. When hydrogen is drawn from the container, the pressure is reduced until it reaches a certain minimum value and the lever opens the dual mixing valve again, starting the hydrogen generation again. The control mechanism can be on/off or proportional.

In the present text reference is made to mechanical means for controlling the metering and mixing of equal volumes of liquid fuel and activator. It will be obvious that the invention is not limited to mechanical means and that alternative control means may be used to the same effect.

**Table 1**

| **Substance** | **MW [g/mole]** | **Water wt. ratio** | **H₂ [% w/w]** |
|---|---|---|---|
| LiH | 7.95 | 2.27 | 7.8% |
| LiAlH₄ | 37.95 | 0.95 | 10.9% |
| LiBH₄ | 21.78 | 1.65 | 13.9% |
| NaH | 24.00 | 0.75 | 4.8% |
| NaAlH₄ | 54.00 | 0.67 | 9.0% |
| NaBH₄ | 37.83 | 0.95 | 10.9% |
| BeH₂ | 11.03 | 3.27 | 8.6% |
| Be(BH₄)₂ | 38.70 | 1.86 | 14.6% |
| MgH₂ | 26.32 | 1.37 | 6.5% |
| Mg(BH₄)₂ | 53.99 | 1.33 | 12.8% |
| CaH₂ | 42.10 | 0.86 | 5.2% |
| Ca(BH₄)₂ | 69.77 | 1.03 | 11.4% |
| AlH₃ | 30.01 | 1.80 | 7.2% |
| Al(BH₄)₃ | 71.51 | 1.51 | 13.5% |

## Claims

1. Inhaler for systemic administration of an active substance, comprising:
- a first reservoir for storing a hydrogen containing fluid,
- a second reservoir for storing an activator fluid,
- a gas compartment,
- a fluid connector, for connecting the first and the second reservoir with the gas compartment in order to allow the hydrogen containing fluid and the activator fluid to flow to the gas compartment in order to mix and to thereby allow the hydrogen to be released from the hydrogen containing fluid into the gas compartment,
- a pre-catalyst mixing chamber,
- a first air inlet, connected to the pre-catalyst mixing chamber in order to allow ambient air to enter the pre-catalyst mixing chamber
- a gas connector, for connecting the gas compartment with the pre-catalyst mixing chamber, in order to allow hydrogen to flow from the gas compartment to the pre-catalyst mixing chamber in order to mix with ambient air from the first air inlet,
- a catalyst chamber with a catalyst for converting the fluid contained in the pre-catalyst chamber into a mixture of warm air and water vapor, wherein the catalyst chamber is connected to the pre-catalyst mixing chamber in order to allow the fluid contained in the pre-catalyst mixing chamber to flow past the catalyst,
- a container for storing an active substance, comprising a contact area for exposing the active substance to a flow,
- a guide for guiding the mixture of warm air and water vapor past the contact area of the container, in order for active substance to be taken up by the mixture of warm air and water vapor and to create an aerosol containing the active substance.

2. Inhaler according to claim 1, wherein the inhaler comprises:
- means for controlling the temperature level of the mixture of warm air and water vapor.

3. Inhaler according to claim 2, wherein the means for controlling the temperature level of the mixture of warm air and water vapor comprises:
- a post-catalyst mixing chamber for mixing the warm air and water vapor of a first temperature level formed in the catalyst chamber with ambient air, in order to form a mixture of warm air and water vapor of a second temperature level, wherein the post-catalyst mixing chamber is provided with a second air inlet to allow air to enter the post-catalyst mixing chamber.

4. Inhaler according to claim 3, wherein the guide is connected with the post-catalyst mixing chamber for guiding the mixture of warm air and water vapor of a second temperature level from the post-catalyst mixing chamber past the contact area of the container.

5. Inhaler according to one of the proceeding claims, wherein the inhaler comprises:
- a first element with the first reservoir for storing a hydrogen containing fluid, the second reservoir for storing an activator fluid, the gas compartment, and the fluid connector, for connecting the first and the second reservoir with the gas compartment,
- a second element adapted to be connected to the first element, with the pre-catalyst mixing chamber, the gas connector, for connecting the gas compartment with the pre-catalyst mixing chamber, the catalyst chamber with a catalyst for converting the fluid contained in the pre-catalyst chamber into a mixture of warm air and water vapor of a first temperature level, and the post-catalyst mixing chamber for mixing the warm air and water vapor of a first temperature level formed in the catalyst chamber with ambient air, in order to form a mixture of warm air and water vapor of a second temperature level form,
- a third element, adapted to be connected to the second element, with the container for storing an active substance, comprising a contact area for exposing the active substance to a flow, the guide connected with the post-catalyst mixing chamber for guiding the mixture of warm air and water vapor of a second temperature level from the post-catalyst mixing chamber past the contact area of the container.

6. Inhaler according to one of the proceeding claims, wherein at least one of the first and second reservoirs for storing a hydrogen containing fluid or for storing an activator fluid are formed as a bag from an elastic material, wherein the interior of the bag is suitable for exerting a pressure on the fluid contained in the reservoir.

7. Inhaler according to claim 6, wherein the bag from an elastic material is adapted to put a pressure on the fluid contained in the reservoir of 0.2 - 0.5 Bar.

8. Inhaler according to claim 6, wherein both the first and second reservoirs are formed as a bag from an elastic material, suitable for exerting a pressure on the fluid contained in the reservoir, wherein the assembly of both reservoirs is contained in a further bag from an elastic material in order to provide an equal pressure on the exterior of both reservoirs.

9. Inhaler according to one of the proceedings claims, wherein the fluid connector, for connecting the first and the second reservoir with the gas compartment is provided with a flow regulator in order to control the amount of hydrogen containing fluid and activator fluid entering the gas compartment.

10. Inhaler according to claim 9, wherein the flow regulator is pressure actuated, wherein the flow regulator is exposed to both ambient pressure and the pressure inside the gas compartment, wherein the opening and closing of the flow regulator is controlled by the difference in pressure between the ambient and the pressure inside the gas compartment.

11. Inhaler according to one of the proceedings claims, wherein the first and second reservoir are positioned inside the gas compartment and wherein the gas compartment is provided with an outlet opening, wherein the outlet opening on one end is connected to the gas connector for connecting the gas compartment with the pre-catalyst mixing chamber, and wherein the outlet opening on the other end is connected to a gas channel in the form of a perforated tube, wherein the gas channel supports the first and second reservoirs and creates a channel between the two, wherein the perforations allow the gas in the gas compartment to enter the interior of the gas channel, in order to flow towards the outlet opening.

12. Inhaler according to one of the proceeding claims, wherein the gas connector for connecting the gas compartment with the pre-catalyst mixing chamber is provided with a moveable stopper for controlling the amount of gas entering the pre-catalyst mixing chamber, wherein the moveable stopper is biased towards a closed position by the action of a spring and wherein the moveable stopper can be displaced towards it's open position by means of an under pressure, created at the downstream end of the moveable stopper.

13. Inhaler according to one of the proceeding claims, wherein the container for storing an active substance, comprises an absorbent element for absorbing the active substance and a spring in order to exert a pressure on the absorbent element in order to forward the active substance towards the surface of the absorbent element.
